# EUROPEAN PATENT APPLICATION

(11) **EP 4 502 134 A1**
(43) Date of publication of application: **05.02.2025**
(21) Application number: 23779223.9
(22) Date of filing: 03.03.2023
(51) Int. Cl.: C12M 3/00, C12Q 1/02, C12M 1/34

(54) **INFORMATION PROCESSING DEVICE, INFORMATION PROCESSING METHOD, AND PROGRAM**

(30) Priority: 31.03.2022 JP 2022059429
(71) Applicant: Sony Group Corporation, Tokyo 108-0075 (JP)
(72) Inventor: MATSUMOTO, Masahiro, Tokyo 108-0075 (JP); SAKATA, Moe, Tokyo 108-0075 (JP); IKEDA, Kenji, Tokyo 108-0075 (JP); NAKAGAWA, Kazuhiro, Tokyo 108-0075 (JP); YAMANE, Kenji, Tokyo 108-0075 (JP)
(74) Representative: D Young & Co LLP
(86) International application number: PCT/JP2023/007939
(87) International publication number: WO 2023/189194

(57) **Abstract**

An information processing device (10) includes a pre-culture information acquisition unit (51), a patient information acquisition unit (52), and a reasoner selection unit (13). The pre-culture information acquisition unit (51) acquires pre-culture information on a cell state of a specimen before culture. The patient information acquisition unit (52) acquires patient information on a donor of the specimen. The reasoner selection unit (13) selects, based on the pre-culture information and the patient information, an optimal reasoner (19) to reason about a culture result of the specimen as an actual reasoner from a plurality of reasoners (19).

## Description

### Field

The present invention relates to an information processing device, an information processing method, and a program.

### Background

New treatments, such as immuno-cell therapy, have been used to treat refractory cancers where it is difficult to completely kill the cancer with normal immune function alone. In such treatments, it is considered to take cells from a patient, culture a group of the cells to a certain ratio, and bring the cells to an intended composition.

### Citation List

### Patent Literature

Patent Literature 1: JP 2011-229413 A

### Summary

### Technical Problem

However, there are various types of cells, each of which is biologically related, and it is difficult to culture a group of cells, while bringing the cells to an intended composition. In addition, since the initial state of a group of cells is different when the cells are taken out from a patient, it is assumed that even if machine learning is performed using normal supervised data, it is difficult to perform culture predictions.

Therefore, the present disclosure proposes an information processing device, an information processing method, and a program that are capable of accurately reasoning about a culture result.

### Solution to Problem

According to the present disclosure, an information processing device is provided that comprises: a pre-culture information acquisition unit configured to acquire pre-culture information on a cell state of a specimen before culture; a patient information acquisition unit configured to acquire patient information on a donor of the specimen; and a reasoner selection unit configured to select, based on the pre-culture information and the patient information, an optimal reasoner to reason about a culture result of the specimen as an actual reasoner from a plurality of reasoners. According to the present disclosure, an information processing method in which an information process of the information processing device is executed by a computer, and a program for causing the computer to execute the information process of the information processing device, are provided.

### Brief Description of Drawings

FIG. 1 is a diagram illustrating an example of a configuration of a culture support system.
FIG. 2 is a diagram illustrating an example of registration information on sample data.
FIG. 3 is a diagram illustrating another example of the registration information on sample data.
FIG. 4 is a diagram illustrating an example of a culture recipe.
FIG. 5 is a diagram illustrating an example of a processing flow regarding selection of a reasoner.
FIG. 6 is a diagram illustrating an example of a processing flow regarding registration of a reasoning result.
FIG. 7 is a diagram illustrating an example of a processing flow regarding retraining.
FIG. 8 is a diagram illustrating an example of a culture device.
FIG. 9 is a diagram illustrating an example of an incubator.
FIG. 10 is a diagram illustrating an example of a measurement device.
FIG. 11 is a diagram illustrating another example of the measurement device.
FIG. 12 is a diagram illustrating an example of a hardware configuration of an information processing device.

### Description of Embodiments

Hereinafter, embodiments of the present disclosure will be described in detail with reference to the drawings. In each of the following embodiments, the same parts are denoted by the same reference signs, and redundant description will be omitted.

Note that the description will be given in the following order.
[1. Culture support system]
[2. Registration information on sample data]
[3. Culture recipe]
[4. Information processing method]
[4-1. Selection of reasoner]
[4-2. Registration of reasoning result]
[4-3. Retraining]
[5. Culture device]
[6. Measurement device]
[7. Hardware configuration example]
[8. Effects]

### [1. Culture support system]

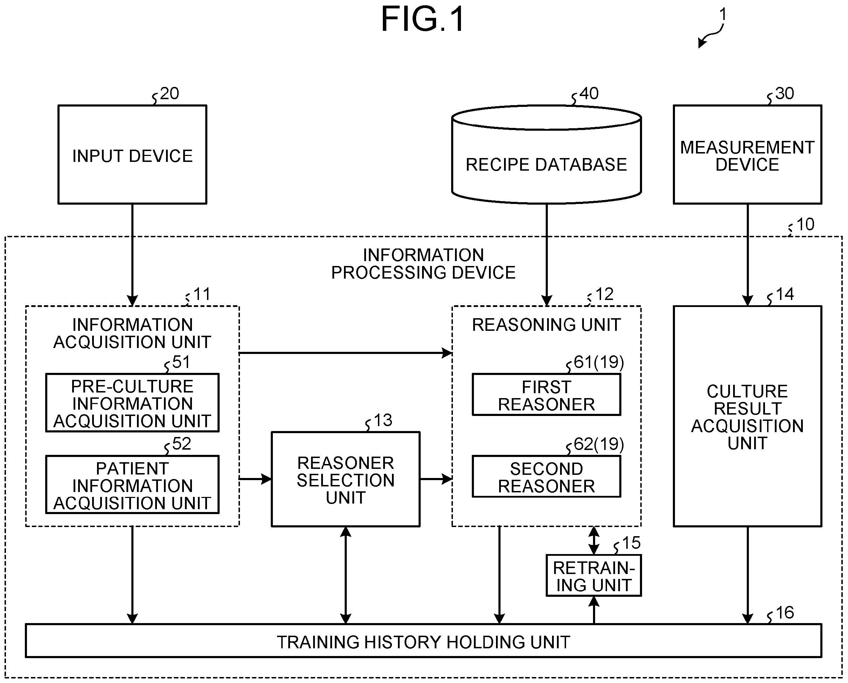

FIG. 1 is a diagram illustrating an example of a configuration of a culture support system 1.

The culture support system 1 presents a highly accurate reasoning result about a culture result of a specimen to support the culture work of medical workers. The culture support system 1 includes an information processing device 10, an input device 20, a measurement device 30, and a recipe database 40.

The input device 20 receives initial information on a specimen before culture. The initial information includes pre-culture information and patient information. The pre-culture information means information on a cell state of the specimen before culture. The patient information means information on a donor (patient) of the specimen. The patient information indicates circumstances (background) behind the patient that are considered to affect the culture result.

For example, the pre-culture information includes information on an RNA expression level, a cell type, a cell composition, or a cell morphology. The cell composition means a ratio of various cells composing the specimen. For example, when the cell type means the type of coreceptor expressed on the surface of T cells (CD8+ T cells, CD4+ T cells, or the like), the cell composition means the composition ratio of a plurality of cells with different types of coreceptors. The cell morphology means the shape and appearance of cells observed under a microscope or the like. The patient information includes information on the age, sex, blood type, race, lifestyle (eating habits, exercise habits, smoking habits, and the like), disease suffered from, administered medicine, or history of past hospital visits of the donor of the specimen. The input device 20 includes a touch panel, a keyboard, a mouse, and the like.

The measurement device 30 measures the specimen before culture and after culture. The measurement result of the specimen before culture is used as the pre-culture information. The measurement result of the specimen after culture is used for pathological diagnosis and for training of a reasoner 19, which will be described later. For example, the culture result includes information on the number of cells, purity (what percentage of desired cells are present relative to the rest of the cells), proliferation rate, cell composition, or survival rate of the specimen after culture. As the measurement device 30, various measurement instruments used in the field of culture are employed. The recipe database 40 stores a plurality of culture recipes with different culture conditions.

The information processing device 10 performs, based on various types of input information and measurement information, processing such as reasoning of the culture result, calculation of reasoning accuracy, and retraining of the reasoner 19 based on the reasoning accuracy. The information processing device 10 includes an information acquisition unit 11, a reasoning unit 12, a reasoner selection unit 13, a culture result acquisition unit 14, a retraining unit 15, and a training history holding unit 16.

The information acquisition unit 11 acquires various types of information from the input device 20. For example, the information acquisition unit 11 includes a pre-culture information acquisition unit 51 that acquires the pre-culture information, and a patient information acquisition unit 52 that acquires the patient information.

The reasoning unit 12 includes a plurality of reasoners 19. Each reasoner 19 uses the pre-culture information, the patient information, and the culture condition as input and the culture result as output. The plurality of reasoners 19 includes a plurality types of trained artificial intelligence (AI) with different learning algorithms. The reasoning unit 12 uses pre-culture information on a cell state of a past sample before culture, patient information on a patient who is a donor of the past sample, and a culture condition of the past sample as input data and a culture result of the past sample as correct data to train the reasoner 19 that reasons about the culture result.

In the example in FIG. 1, the reasoning unit 12 includes a first reasoner 61 and a second reasoner 62. The first reasoner 61 is a reasoner 19 obtained by the decision tree-based Random Forest. The second reasoner 62 is a reasoner 19 obtained by Light Gradient Boosting Machine (GBM) that is reinforcement ensemble training. The number of the reasoners 19 is not limited to two. A support vector machine or the like, which is a classical identifier, may be included as a third reasoner.

The reasoner selection unit 13 selects, based on the pre-culture information and the patient information, an optimal reasoner 19 that should reason about the culture result of the specimen as an actual reasoner from the plurality of reasoners 19. The selection of the reasoner 19 is performed in consideration of information on a past sample registered in the training history holding unit 16. The past sample means a specimen that has been cultured in the past and a culture result has been obtained.

The training history holding unit 16 stores sample data for each past sample. The sample data includes pre-culture information on the past sample, patient information, a culture result, the reasoner 19 that has reasoned about the culture result, and accuracy of reasoning about the culture result. The reasoner selection unit 13 searches for one or more pieces of sample data in which the pre-culture information and/or the patient information is similar to the specimen. The reasoner selection unit 13 selects a reasoner 19 with the highest accuracy of reasoning about the culture result as the actual reasoner from one or more reasoners 19 associated with the one or more pieces of retrieved sample data.

The culture result acquisition unit 14 acquires measurement information indicating the culture result of the specimen from the measurement device 30. The culture result acquisition unit 14 compares the culture result obtained by measurement with a reasoning result by the actual reasoner to calculate the accuracy of reasoning. The training history holding unit 16 acquires, from the information acquisition unit 11, the reasoner selection unit 13, the reasoning unit 12, and the culture result acquisition unit 14, the pre-culture information, the patient information, the reasoner 19 used to reason about the specimen, and the information on the accuracy of reasoning, and registers them as sample data on the specimen.

The retraining unit 15 retrains the reasoners 19 using the newly registered sample data. The retraining is periodically performed for each reasoner 19. For example, the retraining unit 15 extracts one or more pieces of specific sample data associated with a single reasoner 19 from a plurality of pieces of sample data newly registered in the training history holding unit 16. The retraining unit 15 uses the extracted one or more pieces of specific sample data to retrain the reasoner 19 associated with the one or more pieces of specific sample data.

### [2. Registration information on sample data]

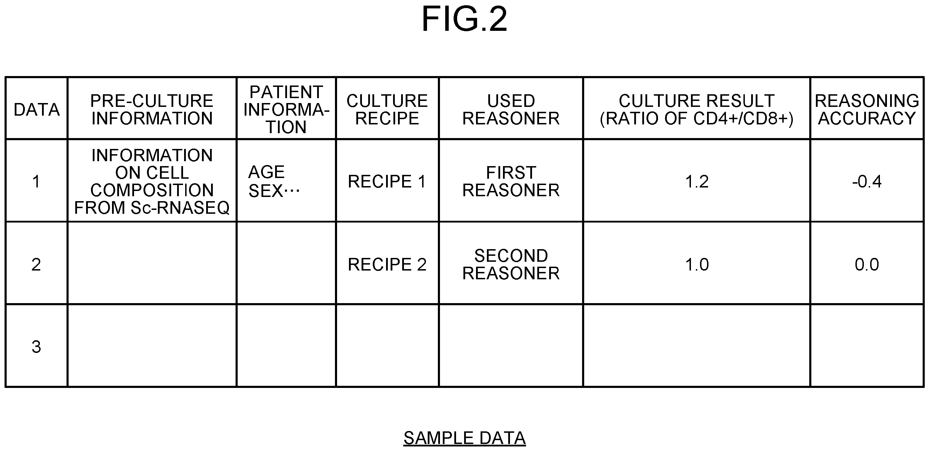

FIG. 2 is a diagram illustrating an example of registration information on sample data.

In the training history holding unit 16, sample data on a plurality of past samples is registered. In FIG. 2, "Data" indicates the numbers of the past samples. The sample data includes the pre-culture information on the corresponding past sample, the patient information, the culture recipe, used reasoner, and the information on the culture result and reasoning accuracy.

For example, the pre-culture information includes information on the initial state of the cells obtained from the observed feature amount before culture. The information obtained by the initial measurement is registered as the pre-culture information. The pre-culture information includes information on an RNA expression level, a cell type, a cell composition, and a cell morphology.

For example, in the initial measurement before culture, 10,000 cells are obtained from the cells to be cultured, and the RNA expression level of each cell is measured using single-cell RNA sequencing. From the measured RNA expression level, the types of cells obtained are determined using Ontology Database or the like. The types of cells are determined using, for example, Cell Ontology-based classification (CellO). Examples of the types of cells include CD4+ T cells, CD8+ T cells, and the like.

The patient information includes information on the background of the specimen considered to affect the culture result. The patient information includes information on the age, sex, race, disease suffered from, administered medicine, and history of past hospital visits of a patient who is the donor of the past sample. The patient information is acquired by a medical inquiry or an interview.

The culture result includes measurement information obtained by measuring the cells of the specimen after culture. The measurement content is determined according to the purpose of culture. For example, information such as the number of cells, purity, proliferation rate, cell composition, and survival rate of the specimen after culture is acquired as the culture result. In the example in FIG. 2, the ratio of CD4+ T cells to CD8+ T cells is registered as the culture result.

The reasoning accuracy includes information on a difference between the culture result (reasoning value) reasoned about by the reasoner 19 and the culture result (measurement value) obtained by measurement after culture. The difference may be calculated as a result of simple subtraction or as a ratio. In the example in FIG. 2, the reasoning accuracy is calculated as the ratio of the difference between the measurement value and the reasoning value to the measurement value.

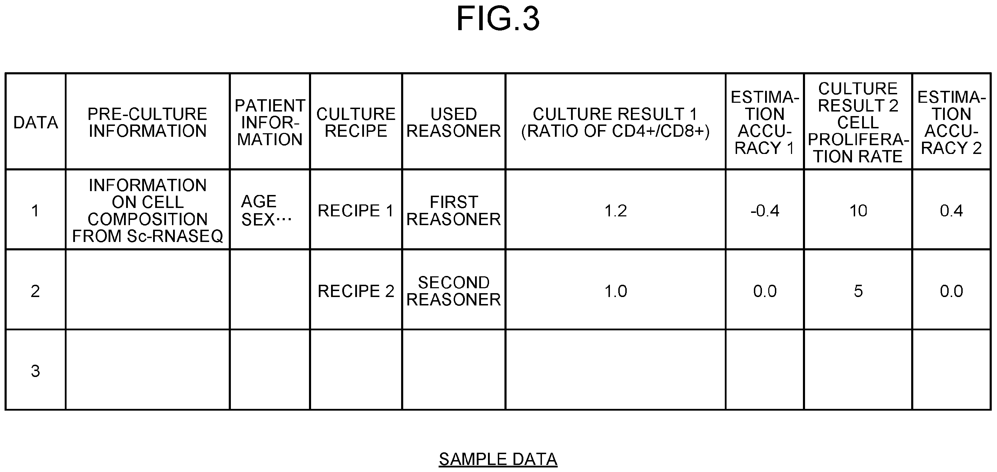

FIG. 3 is a diagram illustrating another example of the registration information on sample data.

In the example in FIG. 2, only one piece of measurement information as the culture result is registered. In the example in FIG. 3, a plurality of pieces of measurement information as the culture result is registered in one piece of sample data.

The training history holding unit 16 stores a plurality of pieces of measurement information as the culture result. The reasoning about each measurement information is performed by a single reasoner 19. The training history holding unit 16 stores the reasoning result and the reasoning accuracy of the reasoner 19 associated with each measurement information. In the example in FIG. 3, the ratio of CD4+ T cells to CD8+ T cells and the cell proliferation rate as the plurality of pieces of measurement information are registered in one piece of sample data.

When a plurality of pieces of measurement information is registered in the sample data, the actual reasoner is selected by preferentially considering the reasoning result of specific measurement information designated by a user. The information acquisition unit 11 acquires information indicating the reasoning result to be prioritized as user input information. The reasoner selection unit 13 selects the reasoner 19 with the highest accuracy of reasoning about the measurement information prioritized based on the user input information among the plurality of pieces of measurement information as the actual reasoner.

### [3. Culture recipe]

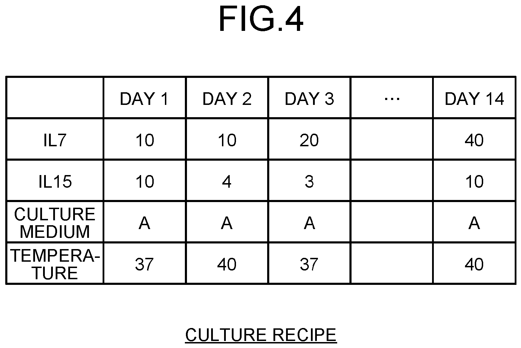

FIG. 4 is a diagram illustrating an example of a culture recipe.

The culture recipe is a table that describes under what conditions cells are cultured (culture conditions). For example, the culture conditions include information on a stimulant to be added to the cells, an amount of the stimulant, a type of culture medium, and a culture temperature. In FIG. 4, interleukin 5 (IL5) and interleukin 7 (IL7) are shown as stimulants.

### [4. Information processing method]

### [4-1. Selection of reasoner]

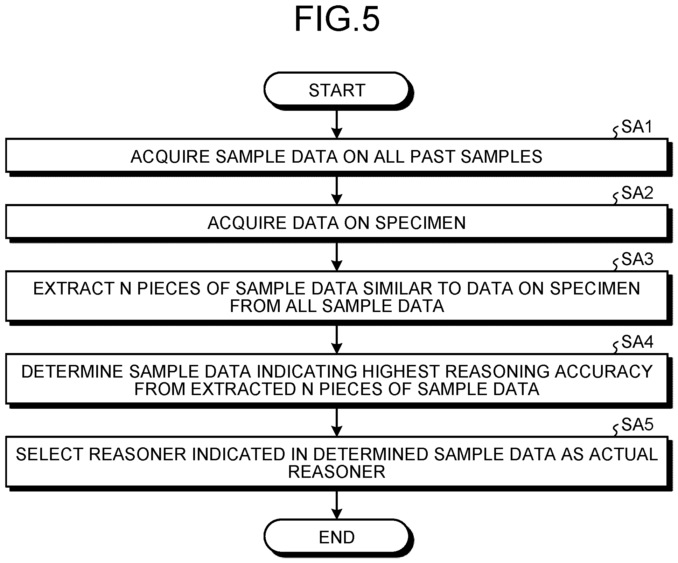

Hereinafter, an example of information processing performed by the information processing device 10 will be described. FIG. 5 is a diagram illustrating an example of a processing flow regarding selection of the reasoner 19.

The reasoner selection unit 13 acquires sample data on all past samples from the training history holding unit 16 (step SA1). The reasoner selection unit 13 acquires the data on the specimen (the pre-culture information, the patient information) (step SA2).

The reasoner selection unit 13 extracts N (N is an integer of 1 or more) pieces of sample data similar to the data on the specimen from all sample data (step SA3). The similarity determination is performed, for example, based on cosine similarity in the case of text information, and based on Euclidean distance in the case of numerical information. By providing a threshold for the cosine similarity or the Euclidean distance, a target number of pieces of sample data is extracted.

The reasoner selection unit 13 determines the sample data indicating the highest reasoning accuracy from the extracted N pieces of sample data (step SA4). The reasoner selection unit 13 selects the reasoner 19 indicated in the determined sample data as the actual reasoner (step SA5). When a plurality of pieces of measurement information is acquired as the culture result and a plurality of pieces of reasoning accuracy is registered in the sample data, the reasoner selection unit 13 selects the reasoner 19 with the highest accuracy of reasoning about the measurement information prioritized based on the user input information as the actual reasoner.

### [4-2. Registration of reasoning result]

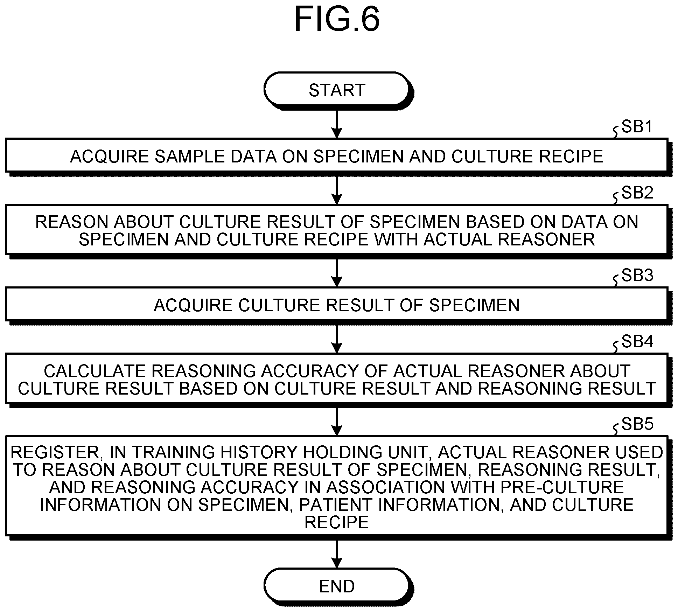

FIG. 6 is a diagram illustrating an example of a processing flow regarding registration of the reasoning result.

The actual reasoner acquires the data on the specimen (the pre-culture information, the patient information) and a culture recipe of the specimen (step SB1). The culture recipe may be designated by the user or may be set by default. The actual reasoner reasons about the culture result of the specimen based on the data on the specimen and the culture recipe (step SB2).

The culture result acquisition unit 14 acquires the culture result of the specimen from the measurement device 30 (step SB3). The culture result acquisition unit 14 calculates the reasoning accuracy of the actual reasoner about the culture result based on the culture result and the reasoning result (step SB4).

The training history holding unit 16 acquires the pre-culture information on the specimen, the patient information, the culture recipe, the actual reasoner, and the information on the reasoning result and the reasoning accuracy from the information acquisition unit 11, the reasoning unit 12, the reasoner selection unit 13, and the culture result acquisition unit 14. The training history holding unit 16 registers the actual reasoner used to reason about the culture result of the specimen, the reasoning result, and the reasoning accuracy in association with the pre-culture information on the specimen, the patient information, and the culture recipe (step SB5).

### [4-3. Retraining]

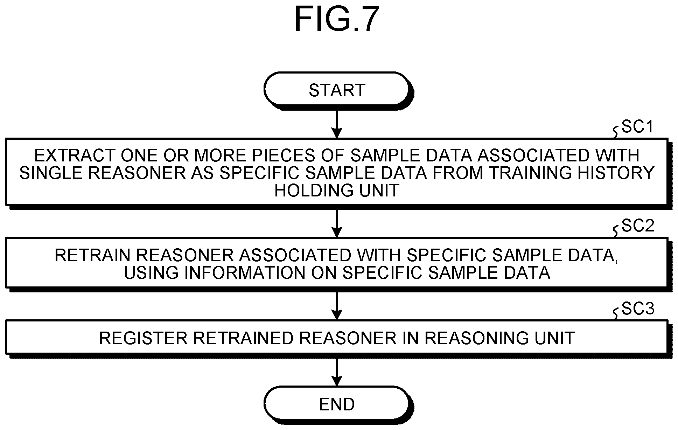

FIG. 7 is a diagram illustrating an example of a processing flow regarding retraining.

The retraining unit 15 extracts a plurality of pieces of sample data newly registered after the previous retraining from the training history holding unit 16. The retraining unit 15 extracts one or more pieces of sample data associated with a single reasoner 19 as specific sample data from the plurality of extracted sample data (step SC1).

The retraining unit 15 retrains the reasoner 19 associated with the specific sample data, using the information in the specific sample data (step SC2). The retraining unit 15 registers the retrained reasoner 19 in the reasoning unit 12 (step SC3).

### [5. Culture device]

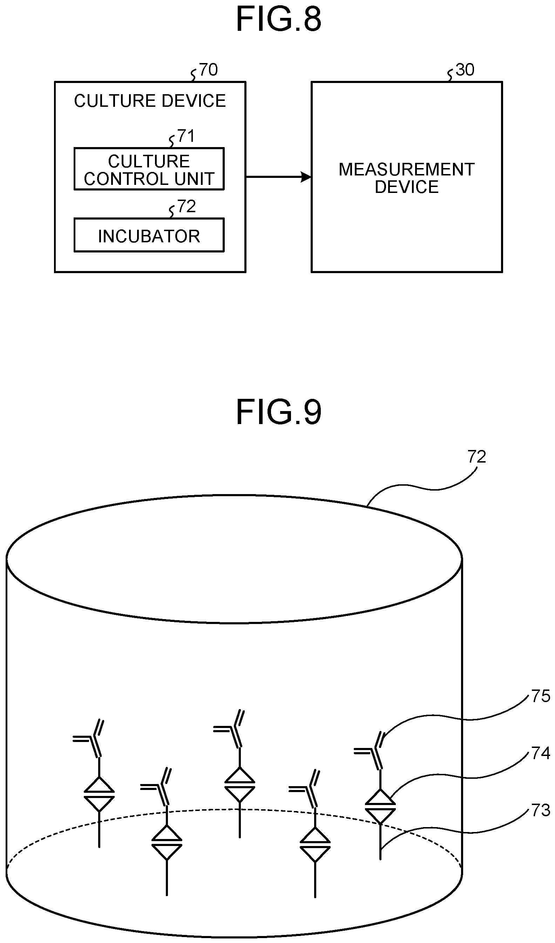

Hereinafter, a specific example of a peripheral device will be described. FIG. 8 is a diagram illustrating an example of a culture device 70.

The culture device 70 cultures a biological sample obtained as the specimen from a patient. The biological sample may be a sample containing a biological component. The biological component may be tissues or cells of a living body, a liquid component of a living body (such as blood or urine), cultures, or living cells (cardiomyocytes, neurons, fertilized eggs, or the like). The biological sample may be a solid, a sample fixed with a fixing reagent such as paraffin, or a solid formed by freezing. The biological sample can be a section of a solid. A specific example of the biological sample includes a section of a biopsy sample.

The biological sample may have been subjected to treatment such as staining or labeling. The treatment may be staining for indicating the morphology of the biological component or the material (such as surface antigens) of the biological component, and examples thereof include Hematoxylin-Eosin (HE) staining and Immunohistochemistry staining. The biological sample may have been subjected to the treatment with one or two or more reagents, and the reagent can be a fluorescent dye, a coloring reagent, a fluorescent protein, or a fluorescence-labeled antibody.

The sample has different properties depending on the type of tissue used (for example, organs or cells), the type of targeted disease, attributes of the patient (for example, age, sex, blood type, race, or the like), the lifestyle of the patient (for example, dietary habits, exercise habits, smoking habits, or the like), and the like. The sample may be managed by attaching identification information (such as barcode information or QR code (registered trademark) information) that can identify each sample.

The culture device 70 includes a culture control unit 71 and an incubator 72. FIG. 9 is a diagram illustrating an example of the incubator 72.

In the incubator 72, a capturing molecule 75 that can bind to a cell to be cultured (target cell) is immobilized via a degradable linker 74. The degradable linker 74 may be immobilized on the bottom surface of the culture vessel. The incubator 72 may be variable so that its volume may be increased or decreased. When the target cell is put into the culture vessel, the incubator 72 captures the target cell with the capturing molecule 75 immobilized therein. The captured target cell is cultured in the culture vessel.

Inside the culture vessel, the capturing molecule 75 is immobilized via, for example, a polymer 73 and the degradable linker 74. The degradable linker 74 may be directly immobilized without the polymer 73. The immobilization is not limited to the bottom surface of the culture vessel and may be on the inner wall. If a planar or three-dimensional structure is present in the culture vessel, the immobilization may be on the surface of the structure. The inner surface of the culture vessel is desirably coated with a substance suitable for cell survival (for example, collagen, fibroblast, or the like).

When the polymer 73 is used, the polymer 73 is preferably one that does not stress the cells or is nontoxic or has biocompatibility. Examples of the polymer 73 include polyethylene glycol (PEG) and a 2-methacryloyloxyethyl phosphorylcholine polymer (MPC polymer).

When the polymer 73 is used, the degradable linker 74 may be bonded to the end opposite to the position where the polymer 73 is bonded to the culture vessel. The degradable linker 74 is a molecule that degrades in response to a specific external stimulus. The degradable linker 74 connects the capturing molecule 75 to the bottom surface of the culture vessel with or without the polymer 73. Examples of the degradable linker 74 include a linker that is degraded by light with a specific wavelength, a linker that is degraded by an enzyme, a linker that is degraded by temperature, and the like. The degradable linker 74 is preferably a photodegradable linker because it can be controlled per single cell and has a short degradation time.

The capturing molecule 75 has a site capable of binding to the cell. As the site capable of binding to the cell, for example, an oleyl group, a cholesteryl group, an antibody, an aptamer, a molecular recognition polymer, or the like can be used. The capturing molecule 75 is preferably immobilized so that one target cell is bound per spot. By capturing one cell per spot, cells (antibodies, sugar chains, or the like) having a molecule capable of binding to the capturing molecule 75 can be sorted at a single cell level. Furthermore, such selection can be performed for all spots.

### [6. Measurement device]

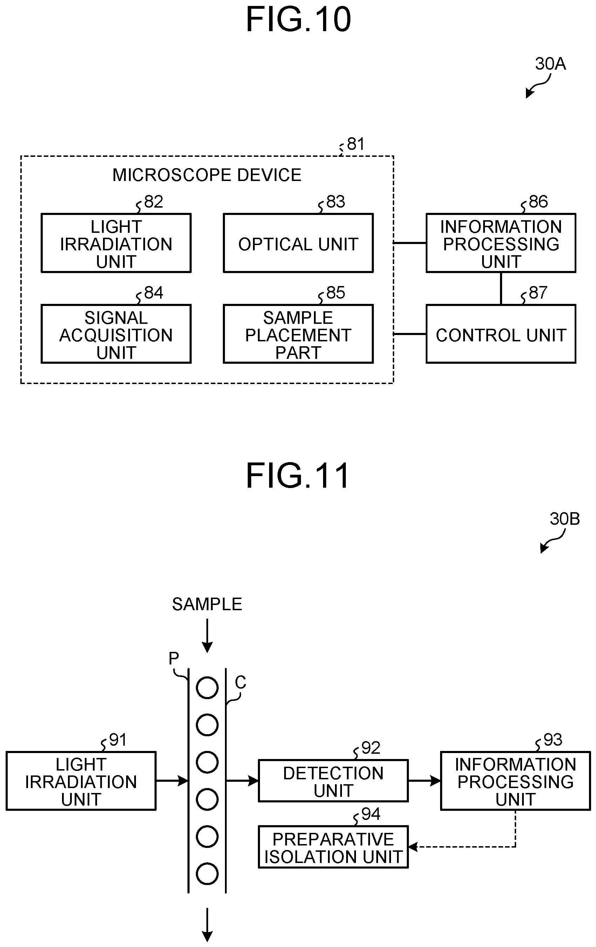

FIG. 10 is a diagram illustrating an example of the measurement device 30. In the example in FIG. 10, the measurement device 30 is configured as a microscope system 30A.

The microscope system 30A includes a microscope device 81, a control unit 87, and an information processing unit 86. The microscope device 81 includes a light irradiation unit 82, an optical unit 83, and a signal acquisition unit 84. The microscope device 81 may further include a sample placement part 85 on which a biological sample is placed. The microscope device 81 may be configured by one or two or more of bright field observation, phase contrast observation, differential interference contrast observation, polarization observation, fluorescence observation, and dark field observation.

The microscope system 30A may be configured as what is called a whole slide imaging (WSI) system or a digital pathology system, and can be used for pathological diagnosis. The microscope system 30A may also be configured as a fluorescence imaging system, particularly a multiple fluorescence imaging system.

The microscope device 81 can acquire data on the biological sample acquired from the patient and transmit the data to the information processing unit 86. The microscope device 81 can transmit the acquired data on the biological sample to the information processing unit 86 located in a place (another room, a building, or the like) away from the microscope device 81. The information processing unit 86 receives the data and outputs the data to the information processing device 10.

The light irradiation unit 82 is a light source that illuminates the biological sample, and an optical unit that guides light emitted from the light source to the sample. The light source can irradiate the biological sample with visible light, ultraviolet light, infrared light, or a combination thereof. The light source may be one or two or more of a halogen lamp, a laser light source, an LED lamp, a mercury lamp, and a xenon lamp. The type and/or wavelength of the light source in fluorescence observation may be plural and may be appropriately selected by those skilled in the art. The light irradiation unit can have a transmissive, reflective, or epi-illumination (coaxial epi-illumination or side-illumination) configuration.

The optical unit 83 is configured to guide the light from the biological sample to the signal acquisition unit 84. The optical unit 83 can be configured to enable the microscope device 81 to observe or image the biological sample.

The signal acquisition unit 84 can be configured to receive the light from the biological sample and convert the light into an electric signal, particularly a digital electric signal. The signal acquisition unit 84 may be configured to acquire data on the biological sample based on the electric signal. The signal acquisition unit 84 may be configured to acquire data on an image of the biological sample (an image, particularly a still image, a time-lapse image, or a moving image), and in particular can be configured to acquire data on an image magnified by the optical unit 83.

The signal acquisition unit 84 includes one or a plurality of imaging elements, CMOS, CCD, or the like, including a plurality of pixels arranged in one or two dimensions. The signal acquisition unit 84 may include an imaging element for acquiring a low-resolution image and an imaging element for acquiring a high-resolution image, or may include an imaging element for sensing for AF or the like and an imaging element for outputting an image for observation or the like.

The imaging element may be a signal processing sensor including, in addition to the plurality of pixels, a signal processing unit (including one, two, or three of a CPU, a DSP, and a memory) that performs signal processing using a pixel signal from each pixel, and an output control unit that controls output of image data generated from the pixel signal and processing data generated by the signal processing unit. Furthermore, the imaging element can include an asynchronous event detection sensor that detects, as an event, that a change in luminance of a pixel that photoelectrically converts incident light exceeds a predetermined threshold. The imaging element including the plurality of pixels, the signal processing unit, and the output control unit can be preferably configured as a one-chip semiconductor device.

The control unit 87 controls imaging by the microscope device 81. The control unit 87 can adjust the positional relationship between the optical unit 83 and the sample placement part 85 by driving the movement of the optical unit 83 and/or the sample placement part 85 to control the imaging. The control unit 87 can move the optical unit 83 and/or the sample placement part 85 in a direction of approaching or separating from each other (for example, an optical axis direction of the objective lens). In addition, the control unit 87 may move the optical unit 83 and/or the sample placement part 85 in any direction on a plane perpendicular to the optical axis direction. The control unit 87 may control the light irradiation unit 82 and/or the signal acquisition unit 84 to control the imaging.

The sample placement part 85 may be configured to fix the position of the biological sample on the sample placement part 85, and may be what is called a stage. The sample placement part 85 can be configured to move the position of the biological sample in the optical axis direction of the objective lens and/or a direction perpendicular to the optical axis direction.

The information processing unit 86 can acquire data (such as imaging data) acquired by the microscope device 81 from the microscope device 81. The information processing unit 86 can perform image processing on the imaging data. The image processing may include color separation processing. The color separation processing may include processing for extracting data on a light component with a predetermined wavelength or wavelength range from the imaging data to generate image data, processing for removing data on a light component with a predetermined wavelength or wavelength range from the imaging data, and the like. In addition, the image processing may include autofluorescence separation processing for separating an autofluorescence component and a dye component of a tissue section, and fluorescence separation processing for separating wavelengths of dyes with different fluorescence wavelengths from each other. In the autofluorescence separation processing, processing for using an autofluorescence signal extracted from one of the plurality of samples having the same or similar properties to remove an autofluorescence component from image information on the other samples may be performed.

The information processing unit 86 may transmit data for controlling imaging to the control unit 87, and the control unit 87 that has received the data may control imaging by the microscope device 81 according to the data. The information processing unit 86 may be configured as an information processing unit such as a general-purpose computer, and may include a CPU, a RAM, and a ROM. The information processing unit 86 may be included in a housing of the microscope device 81 or may be outside the housing. In addition, various types of processing or functions by the information processing unit 86 may be implemented by a server computer or a cloud connected via a network.

Note that the image acquired by the signal acquisition unit 84 may be a stained image and/or an unstained image. The signal acquisition unit 84 may acquire information on the cells before, during, and after processing as the feature amount from the image.

The stained image is, for example, a fluorescence image obtained by irradiating the biological sample stained with a fluorescent reagent with excitation light by the light irradiation unit 82. This makes molecular marker analysis of a biological sample with a biomarker, such as CD4 or CD8, simple and quantitative.

The unstained image may be a bright field image, phase contrast image, or polarized image obtained from an unstained biological sample. Furthermore, the unstained image may be an image that is identified from information learned from the unstained image and the fluorescence image and is pseudo-stained for each cell feature. The pseudo-stained image can be used to predict various labels such as nucleus, cell type (such as nerve), and cell state (such as cell death) from the unstained image and to eliminate the limitation of the number of simultaneous labels due to overlapping fluorescence spectra caused by chemical staining. A specific method regarding the pseudo stained image is not particularly limited as long as it is a known method.

FIG. 11 is a diagram illustrating another example of the measurement device 30. In the example in FIG. 11, the measurement device 30 is configured as a biological sample analyzer 30B.

The biological sample analyzer 30B includes a light irradiation unit 91 that irradiates a biological sample flowing through a flow channel C with light, a detection unit 92 that detects light generated by the irradiation, and an information processing unit 93 that processes information on the light detected by the detection unit 92. Examples of the biological sample analyzer 30B include a flow cytometer and an imaging cytometer. The biological sample analyzer 30B may include a preparative isolation unit 94 that performs a preparative isolation of a specific biological particle P in the biological sample. An example of the biological sample analyzer 30B including the preparative isolation unit 94 can include a cell sorter.

The biological sample may be a liquid sample containing bioparticles. The flow channel C can be configured to allow the biological sample to flow, in particular to form a flow in which the biological particles contained in the biological sample are arranged substantially in a line. A flow channel structure including the flow channel C may be designed to form a laminar flow, and in particular is designed to form a laminar flow in which the flow of the biological sample (sample flow) is enveloped by the sheath liquid flow. The design of the flow channel structure may be appropriately selected by those skilled in the art, and a known channel structure may be employed.

The flow channel C may be formed in a flow channel structure such as a microchip (a chip having a flow channel on the order of micrometers) or a flow cell. The width of the flow channel C is 1 mm or less, and may be particularly 10 µm or more and 1 mm or less. The flow channel C and the flow channel structure including the flow channel C may be formed of a material such as plastic or glass.

The light irradiation unit 91 includes a light source unit that emits light, and a light guide optical system that guides the light to the flow channel C. The light source unit includes one or a plurality of light sources. The type of the light source can be, for example, a laser light source or an LED. The wavelength of the light emitted from each light source may be any wavelength of ultraviolet light, visible light, or infrared light. The light guide optical system includes, for example, an optical component such as a beam splitter group, a mirror group, or an optical fiber. In addition, the light guiding optical system may include a lens group for condensing light, and can include, for example, an objective lens. There may be one or a plurality of points of light irradiation to the biological sample. The light irradiation unit 91 may be configured to condense light emitted from one or a plurality of different light sources with respect to one irradiation point.

The detection unit 92 includes at least one photodetector that detects light generated by irradiating the particles with light by the light irradiation unit 91. The light to be detected is, for example, fluorescence or scattered light (for example, any one or more of forward scattered light, backward scattered light, and side scattered light). Each photodetector includes one or more light receiving elements, for example, a light receiving element array. Each photodetector may include one or a plurality of photomultiplier tubes (PMTs) and/or photodiodes, such as APD and MPPC, as the light receiving elements. The photodetector includes, for example, a PMT array in which a plurality of PMTs is arranged in a one-dimensional direction. The detection unit 92 may further include an imaging element such as a CCD or a CMOS. The detection unit can acquire an image (for example, a bright-field image, a dark-field image, a fluorescence image, and the like) of the bioparticles by the imaging element.

The detection unit 92 can include a signal processing unit that converts an electrical signal obtained by the photodetector into a digital signal. The signal processing unit may include an A/D converter as a device that performs the conversion. The digital signal obtained by the conversion by the signal processing unit can be transmitted to the information processing unit 93. The digital signal can be handled as data on light (hereinafter, also referred to as "light data") by the information processing unit 93. The light data may be, for example, light data including fluorescence data. More specifically, the light data may be light intensity data, and the light intensity may be light intensity data on light including fluorescence (feature quantities of Area, Height, Width, and the like may be included).

The information processing unit 93 includes, for example, a processing unit that processes various types of data (for example, the light data) and a storage unit that stores various types of data. When acquiring the light data corresponding to the fluorescent dye from the detection unit 92, the processing unit can perform fluorescence leakage correction (compensation processing) on the light intensity data. In addition, in the case of the spectral-type flow cytometer, the processing unit performs fluorescence separation processing on the light data and acquires light intensity data corresponding to the fluorescent dye. When the detection unit 92 includes an imaging element, the processing unit may acquire morphology information on the bioparticles based on the image acquired by the imaging element. The storage unit may be configured to store the acquired light data. The storage unit may be configured to further store spectral reference data to be used in unmixing processing.

When the biological sample analyzer 30B includes the preparative isolation unit 94, the information processing unit 93 can determine whether to perform a preparative isolation of the biological particles based on the light data and/or the morphology information. Then, the information processing unit 93 controls the preparative isolation unit 94 based on the result of the determination, and the preparative isolation unit 94 can perform the preparative isolation of the biological particles.

The information processing unit 93 may be configured to output various types of data (for example, light data and images). For example, the information processing unit 93 can output various types of data (for example, two-dimensional plots, spectral plots, and the like) generated based on the light data to the information processing device 10. In addition, the information processing unit 93 may be configured to accept input of various types of data, and accept gating processing on a plot by the user, for example. The information processing unit 93 can include an output unit (for example, a display or the like) or a user interface (for example, a keyboard or the like) for performing the output or the input.

The information processing unit 93 may be configured as a general-purpose computer, and may be configured as an information processing unit including, for example, a CPU, a RAM, and a ROM. The information processing unit 93 may be included in a housing in which the light irradiation unit 91 and the detection unit 92 are provided, or may be outside the housing. In addition, various types of processing or functions by the information processing unit 93 may be implemented by a server computer or a cloud connected via a network.

The preparative isolation unit 94 can perform the preparative isolation of the bioparticles according to the result of the determination by the information processing unit 93. The method of preparative isolation may be a method in which droplets containing biological particles are generated by vibration, charges are applied to the droplets to be subjected to preparative isolation, and the direction of movement of the droplets is controlled by an electrode. The method of preparative isolation may be a method in which the direction of movement of the biological particles is controlled in a flow channel structure to perform a preparative isolation. The flow channel structure is provided with, for example, a control mechanism by pressure (injection or suction) or charge.

### [7. Hardware configuration example]

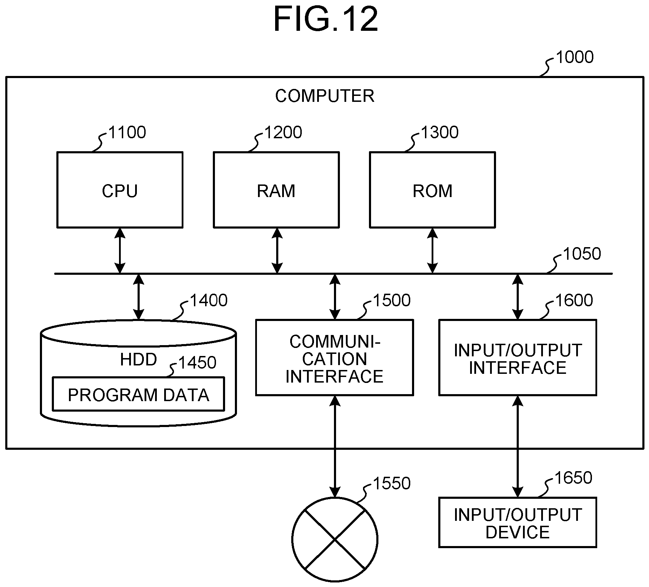

FIG. 12 is a diagram illustrating an example of a hardware configuration of the information processing device 10.

The information processing of the information processing device 10 is implemented by, for example, a computer 1000. The computer 1000 includes a central processing unit (CPU) 1100, a random access memory (RAM) 1200, a read only memory (ROM) 1300, a hard disk drive (HDD) 1400, a communication interface 1500, and an input/output interface 1600. Each unit of the computer 1000 is connected by a bus 1050.

The CPU 1100 operates based on a program (program data 1450) stored in the ROM 1300 or the HDD 1400, and controls each unit. For example, the CPU 1100 loads programs stored in the ROM 1300 or the HDD 1400 into the RAM 1200 to perform processing corresponding to the various programs.

The ROM 1300 stores a boot program such as a basic input output system (BIOS) to be executed by the CPU 1100 when the computer 1000 is activated, a program depending on the hardware of the computer 1000, and the like.

The HDD 1400 is a non-transitory computer-readable recording medium that non-transiently records a program to be executed by the CPU 1100, data to be used by the program, and the like. Specifically, the HDD 1400 is a recording medium that records an information processing program according to the embodiment as an example of the program data 1450.

The communication interface 1500 is an interface for the computer 1000 to connect to an external network 1550 (for example, the Internet). For example, the CPU 1100 receives data from another device and transmits data generated by the CPU 1100 to another device via the communication interface 1500.

The input/output interface 1600 is an interface for connecting an input/output device 1650 and the computer 1000. For example, the CPU 1100 receives data from an input device such as a keyboard or a mouse via the input/output interface 1600. In addition, the CPU 1100 transmits data to an output device such as a display device, a speaker, or a printer via the input/output interface 1600. In addition, the input/output interface 1600 may function as a media interface that reads a program or the like recorded in a predetermined recording medium (medium). The medium is, for example, an optical recording medium such as a digital versatile disc (DVD) or a phase change rewritable disk (PD), a magneto-optical recording medium such as a magneto-optical disk (MO), a tape medium, a magnetic recording medium, a semiconductor memory, or the like.

For example, when the computer 1000 functions as the information processing device 10 according to the embodiment, the CPU 1100 of the computer 1000 implements the functions of the above units by executing the information processing program loaded on the RAM 1200. In addition, the HDD 1400 stores the information processing program according to the present disclosure, various models, and various types of data. Note that the CPU 1100 reads the program data 1450 from the HDD 1400 and executes the program data 1450, but may acquire these programs from another device via the external network 1550, as another example.

### [8. Effects]

The information processing device 10 includes the pre-culture information acquisition unit 51, the patient information acquisition unit 52, and the reasoner selection unit 13. The pre-culture information acquisition unit 51 acquires pre-culture information on the cell state of a specimen before culture. The patient information acquisition unit 52 acquires patient information on a donor of the specimen. The reasoner selection unit 13 selects, based on the pre-culture information and the patient information, an optimal reasoner 19 that should reason about the culture result of the specimen as an actual reasoner from the plurality of reasoners 19. In the information processing method in the present disclosure, the processing of the information processing device 10 is performed by the computer 1000. The program in the present disclosure causes the computer 1000 to perform the processing of the information processing device 10.

According to this configuration, the culture result is accurately reasoned based on the initial information (the pre-culture information, patient information) on the specimen before culture.

The information processing device 10 includes the training history holding unit 16. The training history holding unit 16 stores sample data for each past sample. The sample data includes the pre-culture information on the past sample, the patient information, the culture result, a reasoner that has reasoned about the culture result, and accuracy of reasoning about the culture result. The reasoner selection unit 13 searches for one or more pieces of sample data in which the pre-culture information and/or the patient information is similar to the specimen. The reasoner selection unit 13 selects a reasoner 19 with the highest accuracy of reasoning about the culture result as the actual reasoner from one or more reasoners 19 associated with the one or more pieces of retrieved sample data.

According to this configuration, an appropriate actual reasoner is selected based on the data on the past reasoning accuracy.

The plurality of reasoners 19 includes a plurality of types of trained AI with different learning algorithms using the pre-culture information, the patient information, and the culture condition as input and the culture result as output.

According to this configuration, by providing variations in the learning algorithm, high reasoning accuracy can be obtained for various situations.

The information processing device 10 includes the retraining unit 15. The retraining unit 15 extracts one or more pieces of specific sample data associated with a single reasoner 19 from a plurality of pieces of sample data newly registered in the training history holding unit 16. The retraining unit retrains the reasoner 19 associated with the one or more pieces of specific sample data, using the extracted one or more pieces of specific sample data.

According to this configuration, retraining is easily performed for the culture situation matching the learning algorithm. By repeating the retraining, a good culture situation is created for each reasoner 19, and the respective reasoners 19 grow into reasoners 19 specialized for different culture situations.

The training history holding unit 16 stores a plurality of pieces of measurement information as the culture result. The reasoner selection unit 13 selects the reasoner 19 with the highest accuracy of reasoning about the measurement information prioritized based on the user input information among the plurality of pieces of measurement information as the actual reasoner.

According to this configuration, high reasoning accuracy can be obtained for the culture result of interest.

The pre-culture information includes information on an RNA expression level, a cell type, a cell composition, or a cell morphology. The patient information includes information on an age, a sex, a blood type, a race, a lifestyle, a disease suffered from, an administered medicine, or a history of past hospital visits of the donor of the specimen.

According to this configuration, the features inherent in the specimen can be appropriately reflected in the estimation result.

The culture result includes information on the number of cells, purity, a proliferation rate, a cell composition, or a survival rate of the specimen after culture.

According to this configuration, information such as a cell composition with higher accuracy than before is obtained.

Note that the effects described in the present specification are merely examples and are not limited, and other effects may be provided.

### [Supplementary note]

Note that the present technique can also have the following configurations.
(1) An information processing device comprising:
   a pre-culture information acquisition unit configured to acquire pre-culture information on a cell state of a specimen before culture;
   a patient information acquisition unit configured to acquire patient information on a donor of the specimen; and
   a reasoner selection unit configured to select, based on the pre-culture information and the patient information, an optimal reasoner to reason about a culture result of the specimen as an actual reasoner from a plurality of reasoners.
(2) The information processing device according to (1), further comprising:
   a training history holding unit configured to store, for each past sample, sample data including the pre-culture information, the patient information, the culture result, a reasoner having reasoned about the culture result, and accuracy of reasoning about the culture result, wherein
   the reasoner selection unit searches for one or more pieces of sample data in which the pre-culture information and/or the patient information is similar to the specimen, and selects a reasoner with the highest accuracy of reasoning about the culture result as the actual reasoner from one or more reasoners associated with the one or more pieces of sample data.
(3) The information processing device according to (2), wherein
   the plurality of reasoners includes a plurality of types of trained AI with different learning algorithms using the pre-culture information, the patient information, and a culture condition as input and the culture result as output.
(4) The information processing device according to (3), further comprising:
   a retraining unit configured to extract one or more pieces of specific sample data associated with a single reasoner from a plurality of pieces of sample data newly registered in the training history holding unit and to retrain the reasoner associated with the one or more pieces of specific sample data, using the extracted one or more pieces of specific sample data.
(5) The information processing device according to any one of (2) to (4), wherein
   the training history holding unit stores a plurality of pieces of measurement information as the culture result, and
   the reasoner selection unit selects, as the actual reasoner, a reasoner with the highest accuracy of reasoning about measurement information prioritized based on user input information among the plurality of pieces of measurement information.
(6) The information processing device according to any one of (1) to (5), wherein
   the pre-culture information includes information on an RNA expression level, a cell type, a cell composition, or a cell morphology.
(7) The information processing device according to any one of (1) to (6), wherein
   the patient information includes information on an age, a sex, a blood type, a race, a lifestyle, a disease suffered from, an administered medicine, or a history of past hospital visits of the donor.
(8) The information processing device according to any one of (1) to (7), wherein
   the culture result includes information on the number of cells, purity, a proliferation rate, a cell composition, or a survival rate of the specimen after culture.
(9) An information processing method to be executed by a computer, the method comprising:
   acquiring pre-culture information on a cell state of a specimen before culture;
   acquiring patient information on a donor of the specimen; and
   selecting, based on the pre-culture information and the patient information, an optimal reasoner to reason about a culture result of the specimen as an actual reasoner from a plurality of reasoners.
(10) An information processing method to be executed by a computer, the method comprising:
   using pre-culture information on a cell state of a past sample before culture, patient information on a patient being a donor of the past sample, and a culture condition of the past sample as input data and a culture result of the past sample as correct data to retrain a reasoner configured to reason about the culture result.
(11) A program causing a computer to execute:
   acquiring pre-culture information on a cell state of a specimen before culture;
   acquiring patient information on a donor of the specimen; and
   selecting, based on the pre-culture information and the patient information, an optimal reasoner to reason about a culture result of the specimen as an actual reasoner from a plurality of reasoners.

### Reference Signs List

10 INFORMATION PROCESSING DEVICE
13 REASONER SELECTION UNIT
15 RETRAINING UNIT
16 TRAINING HISTORY HOLDING UNIT
19 REASONER
51 PRE-CULTURE INFORMATION ACQUISITION UNIT
52 PATIENT INFORMATION ACQUISITION UNIT

## Claims

1. An information processing device comprising:
a pre-culture information acquisition unit configured to acquire pre-culture information on a cell state of a specimen before culture;
a patient information acquisition unit configured to acquire patient information on a donor of the specimen; and
a reasoner selection unit configured to select, based on the pre-culture information and the patient information, an optimal reasoner to reason about a culture result of the specimen as an actual reasoner from a plurality of reasoners.

2. The information processing device according to claim 1, further comprising:
a training history holding unit configured to store, for each past sample, sample data including the pre-culture information, the patient information, the culture result, a reasoner having reasoned about the culture result, and accuracy of reasoning about the culture result, wherein
the reasoner selection unit searches for one or more pieces of sample data in which the pre-culture information and/or the patient information is similar to the specimen, and selects a reasoner with the highest accuracy of reasoning about the culture result as the actual reasoner from one or more reasoners associated with the one or more pieces of sample data.

3. The information processing device according to claim 2, wherein
the plurality of reasoners includes a plurality of types of trained AI with different learning algorithms using the pre-culture information, the patient information, and a culture condition as input and the culture result as output.

4. The information processing device according to claim 3, further comprising:
a retraining unit configured to extract one or more pieces of specific sample data associated with a single reasoner from a plurality of pieces of sample data newly registered in the training history holding unit and to retrain the reasoner associated with the one or more pieces of specific sample data, using the extracted one or more pieces of specific sample data.

5. The information processing device according to claim 2, wherein
the training history holding unit stores a plurality of pieces of measurement information as the culture result, and
the reasoner selection unit selects, as the actual reasoner, a reasoner with the highest accuracy of reasoning about measurement information prioritized based on user input information among the plurality of pieces of measurement information.

6. The information processing device according to claim 1, wherein
the pre-culture information includes information on an RNA expression level, a cell type, a cell composition, or a cell morphology.

7. The information processing device according to claim 1, wherein
the patient information includes information on an age, a sex, a blood type, a race, a lifestyle, a disease suffered from, an administered medicine, or a history of past hospital visits of the donor.

8. The information processing device according to claim 1, wherein
the culture result includes information on the number of cells, purity, a proliferation rate, a cell composition, or a survival rate of the specimen after culture.

9. An information processing method to be executed by a computer, the method comprising:
acquiring pre-culture information on a cell state of a specimen before culture;
acquiring patient information on a donor of the specimen; and
selecting, based on the pre-culture information and the patient information, an optimal reasoner to reason about a culture result of the specimen as an actual reasoner from a plurality of reasoners.

10. An information processing method to be executed by a computer, the method comprising:
using pre-culture information on a cell state of a past sample before culture, patient information on a patient being a donor of the past sample, and a culture condition of the past sample as input data and a culture result of the past sample as correct data to retrain a reasoner configured to reason about the culture result.

11. A program causing a computer to execute:
acquiring pre-culture information on a cell state of a specimen before culture;
acquiring patient information on a donor of the specimen; and
selecting, based on the pre-culture information and the patient information, an optimal reasoner to reason about a culture result of the specimen as an actual reasoner from a plurality of reasoners.
